# EUROPÄISCHE PATENTSCHRIFT

(11) **EP 0 641 310 B1**
(45) Veröffentlichungstag und Bekanntmachung des Hinweises auf die Patenterteilung: **15.01.1997**
(21) Anmeldenummer: 93909959.4
(22) Anmeldetag: 12.05.1993
(51) Int. Cl.: C07C 227/00, C07C 229/16

(54) **VERFAHREN ZUR HERSTELLUNG VON $g(b)-ALANINDIESSIGSÄURE ODER IHREN ALKALIMETALL- ODER AMMONIUMSALZEN**
PROCESS FOR PRODUCING BETA-ALANINE DIACETIC ACID OR ITS ALKALI METAL OR AMMONIUM SALTS
PROCEDE DE FABRICATION D'ACIDE $g(b)-ALANINE-DIACETIQUE OU DE SES SELS DE METAUX ALCALINS OU D'AMMONIUM

(30) Priorität: 20.05.1992 DE 4216560
(43) Veröffentlichungstag der Anmeldung: 08.03.1995
(73) Patentinhaber: BASF Aktiengesellschaft, 67063 Ludwigshafen (DE)
(72) Erfinder: KNEIP, Michael, D-6710 Frankenthal (DE); SCHNEIDER, Juergen, D-6713 Freinsheim (DE); OFTRING, Alfred, D-6702 Bad Duerkheim (DE)
(86) Internationale Anmeldenummer: EP9301171
(87) Internationale Veröffentlichungsnummer: WO9323363

(56) Entgegenhaltungen:
- EP-A- 0 356 972
- DE-A- 2 727 755
- CHEMICAL ABSTRACTS, vol. 62, no. 13, 21. Juni 1965, Columbus, Ohio, US; abstract no. 16232a, V. G. YASHUNSKII 'Synthesis of some sydnonimine derivatives' Spalte 16232; in der Anmeldung erwähnt siehe Zusammenfassung & ZH. VSES. KHIM. OBSHCHESTVA IM. D. I. MENDELEEVA Bd. 10, Nr. 1, Seiten 150-156

## Beschreibung

Die vorliegende Erfindung betrifft ein neues Verfahren zur Herstellung von β-Alanindiessigsäure (Ia) oder ihren Alkalimetall- oder Ammoniumsalzen (Ib).

Die Verbindungen Ia und Ib sind ebenso wie die Nitrilotriessigsäure oder die Ethylendiamintetraessigsäure bzw. die Salze dieser Aminopolycarbonsäuren als Komplexbildner von Bedeutung.

Es ist bekannt, β-Alanindiessigsäure durch Umsetzung von Chloressigsäure mit β-Alanin herzustellen (G. Schwarzenbach et al., Helv. Chim. Acta 32 (1949), S. 1184). Weiterhin sind die Cyanomethylierung von β-Alanin zum β-Alanindiacetonitril (V.G. Yashunskii et al., Zh. Vses. Khim. Obshchestva im. D.I. Mendeleeva 10 (1965), S. 105, zitiert in Chem. Abstr. 62 (1965), 16232a) und die Umsetzung von Iminodiessigsäure mit Acrylamid zum β-Alanin-N,N-diessigsäure-monoamid (DE-A 27 27 755) bekannt.

Die beiden erstgenannten Verfahren benötigen das relativ schwer zugängliche β-Alanin als Ausgangsstoff und erfordern mehrere Verfahrensstufen, während die Addition der aus Ammoniak, Blausäure und Formaldehyd leicht erhältlichen Iminodiessigsäure an Acrylamid, ebenso wie die Cyanomethylierung des β-Alanins, nur ein Derivat der β-Alanindiessigsaure liefert, aus dem die Säure erst durch anschließende Hydrolyse der Cyanogruppe bzw. der Amidgruppe erhalten wird.

Aus der DE-OS 38 29 859 ist weiterhin ein Verfahren zur Herstellung von β-Alanindiessigsäure durch Umsetzung von Iminodiessigsäure mit Acrylsäure bekannt.

Da β-Alanindiessigsäure und ihre Salze hauptsächlich als Komplexbildner, beispielsweise in Wasch- und Reinigungsmitteln oder in der Photoindustrie, aber auch in kosmetischen und pharmazeutischen Zubereitungen Verwendung finden, sind hohe Reinheiten erwünscht und erforderlich.

Die equimolare Umsetzung von Iminodiessigsäure bzw. deren Salzen mit Acrylsäure verläuft zwar mit hohen Ausbeuten (bis zu 98 % lt. DE-OS 38 29 859). Die erhaltenen Produkte enthalten aber immer noch unumgesetzte Acrylsäure und/oder Iminodiessigsäure bzw. deren Salze, die sich nur unter Ausbeuteverlust, z.B. durch Umkristallisieren oder durch aufwendige Extraktionsverfahren, entfernen lassen. Aus dem gleichen Grund verbietet es sich natürlich einen Überschuß eines Ausgangsstoffes einzusetzen. Auch bei den anderen Verfahren entstehen bei den angewendeten Temperaturen (≥ 60°C) aufgrund der Reversibilität der Michael-Addition von Iminodiessigsäure an Acrylsäure ebenfalls Iminodiessigsäure und Acrylsäure.

Der vorliegenden Erfindung lag daher die Aufgabe zugrunde, die Titelverbindungen Ia und Ib durch ein einfaches und wirtschaftliches Verfahren ohne spezielle Reinigungsschritte so rein herzustellen, daß sie weniger als 0,5 % Iminodiessigsäure und weniger als 0,1 % Acrylsäure im Endprodukt enthalten.

Demgemäß wurde ein Verfahren zur Herstellung von β-Alanindiessigsäure (Ia) oder ihren Alkalimetall- oder Ammoniumsalzen (Ib) gefunden, welches dadurch gekennzeichnet ist, daß man Iminodiessigsäure mit Acrylnitril oder C₁- bis C₄-Alkylacrylsaureestern im schwach sauren bis schwach basischen wäßrigen Medium miteinander umsetzt und anschließend die Nitril- oder Estergruppe zur Säure oder einem Salz verseift.

Die eingesetzte Iminodiessigsäure oder deren Salze, insbesondere das Natrium- oder Kaliumsalz, sollten eine Mindestreinheit von ca. 95 Gew.-%, vorzugsweise von 99 Gew.-%, aufweisen, da sonst die Reinheit des Endproduktes beeinträchtigt werden könnte. Derart reine Iminodiessigsäure oder Salze sind aber - auch im technischen Maßstab - gut zugänglich.

Das verwendete Acrylnitril bzw. der Acrylester sind wie üblich stabilisiert, z.B. mit 50 ppm Hydrochinonmonomethylether oder Hydrochinon.

Die Reaktionspartner Iminodiessigsäure und Acrylester bzw. Acrylnitril werden zweckmäßigerweise im molaren Verhältnis 1:0,95 bis 1:2, vorzugsweise 1:1,0 bis 1:1,5, eingesetzt.

Lösungsmittel ist in der Regel Wasser. Es kann jedoch bei der Umsetzung von Acrylestern auch ein Gemisch aus Wasser und Alkohol, vorzugsweise der entsprechende Alkohol des verwendeten Acrylesters, eingesetzt werden. Ein Anteil des Alkohols am Lösungsmittelgemisch von 20 Gew.-% ist dabei ausreichend.

Der Gewichtsanteil der Iminodiessigsäure in der gesamten wäßrigen Mischung beträgt im allgemeinen 10 bis 40, bevorzugt 20 bis 30 Gew.-%. Die Addition des Acrylesters bzw. des Acrylnitrils an die Iminodiessigsäure setzt schon unter 0°C deutlich ein und nimmt bei 20 bis 40°C in der Regel 0,25 bis 5 Stunden in Anspruch. Vorzugsweise hält man Temperaturen unter 60°C ein.

pH-Werte von 5 bis 11, vorzugsweise 6 bis 8 sind besonders geeignet.

Nach der Umsetzung wird zweckmäßigerweise ein Luft- oder Inertgasstrom, z.B. Stickstoff, durch die Reaktionsmischung geleitet, um überschüssigen Acrylester bzw. Acrylnitril abzudestillieren. Der abdestillierte Anteil kann für weitere Umsetzungen wieder eingesetzt werden.

Beim Abdestillieren arbeitet man vorteilhafterweise bei einem vermindertem Druck von 100 bis 1000 mbar. Die Temperatur sollte auch hierbei 60°C nicht überschreiten.

Hieran anschließend erfolgt - ohne Isolierung der Zwischenprodukte - die Verseifung des β-Alanindfessigsäure-esters bzw. -nitrils in alkalischem oder Stickstoff basischem wäßrigem Medium oder in wäßrig mineral saurem Medium. Der pH-Wert der wäßrigen Lösung kann demgemäß im Bereich von 0 bis 14 liegen, insbesondere liegt er bei 4 bis 9. Nötigenfalls wird er mit einer Mineralsäure, beispielsweise Schwefelsäure, Salzsäure, ortho-Phosphorsäure oder Salpetersäure bei der sauren Verseifung, bzw. mit wäßrigem Alkali, beispielsweise Natronlauge, Kalilauge, Natriumhydrogencarbonat oder Natriumcarbonat oder einer Stickstoffbase wie Ammmoniak oder Triethylamin bei der alkalischen Verseifung eingestellt oder konstant gehalten. Verseifungstemperaturen von 20 bis 60°C, insbesondere 40 bis 60°C sind zweckmäßig. Unter diesen schonenden Verseifungsbedingungen werden Rückspaltungen nicht beobachtet. Die Reaktion ist bei Temperaturen von 40 bis 60°C in der Regel in 5 bis 24 Stunden beendet.

Weiterhin ist es günstig, einen Luft- oder Inertgasstrom, z.B. Stickstoff, während der Verseifung durch die Reaktionsmischung zu leiten und gebildeten Alkohol bzw. Ammoniak in Form der wäßrigen Lösungen kontinuierlich abzudestillieren.

Falls gewünscht können die Alkalimetall- bzw. Ammoniumsalze Ib auf an sich bekannte Weise, z.B. durch Abdestillieren von Wasser und anschließende Kristallisation oder durch Sprühtrocknung, in fester Form erhalten werden.

Sollen ein, zwei oder vorzugsweise drei Carboxylgruppen von Ib als freie Säure vorliegen, werden diese durch Zugabe der entsprechenden Menge Mineralsäure aus ihren Salzen freigesetzt. Als Mineralsäuren können beispielsweise Schwefelsäure, Salzsäure, ortho-Phosphorsäure oder Salpetersäure verwendet werden. Als besonders günstig hat sich 20 bis 100 gew.-%ige, insbesondere 30 bis 70 gew.-%ige Schwefelsäure erwiesen.

Man arbeitet dabei in der Regel bei Temperaturen von 10 bis 60°C, vorzugsweise 20 bis 50°C. Die freie Carboxylgruppen enthaltenden Verbindungen I werden zweckmäßigerweise durch Kristallisation oder Fällung isoliert. Soll β-Alanindiessigsaure mit drei freien Carboxylgruppen erhalten werden, fällt man das Produkt besonders vorteilhaft durch Zugabe von 30 bis 70 gew.-%iger Schwefelsaure bei einem pH-Wert von 1,5 bis 2,5, insbesondere 1,8 bis 2,2 aus. Es empfiehlt sich, die Fällung oder Kristallisation durch anschließendes Abkühlen auf Temperaturen von 0 bis 35°C zu vervollständigen.

Das erfindungsgemäße Verfahren kann sowohl diskontinuierlich als auch kontinuierlich durchgeführt werden.

Gegenstand der vorliegenden Erfindung ist weiterhin die Verwendung der nach dem erfindungsgemäßen Verfahren hergestellten β-Alanindiessigsäure und ihrer Alkalimetallsalze I als Komplexbildner.

Das erfindungsgemäße Verfahren gestattet es, β-Alanindiessigsäure und ihre Alkalimetallsalze in hochreiner Form, d.h. mit höheren Gehalten als 99,5 Gew.-% und mit weniger als 0,5 Gew.-% Iminodiessigsäure und weniger als 0,1 Gew.-% Acrylsäure, ohne zusätzliche Reinigungsschritte in einer einfachen und wirtschaftlichen Synthese auch im technischen Maßstab herzustellen.

Das erfindungsgemäße Verfahren liefert zudem keine störenden Nebenprodukte, anfallendes Acrylnitril bzw. Acrylester und eventuell anfallender Alkohol können wieder eingesetzt werden und anfallendes Ammoniakwasser kann leicht einer weiteren Verwertung zugeführt werden.

### Beispiel 1

### Herstellung von β-Alanindiessigsäure-tri-Na-Salz (ADA-Na₃) (Acrylnitril-Weg)

Zu 3540 g einer 40 gew.-%igen wäßrigen Lösung des Dinatriumsalzes von Iminodiessigsäure (entsprechend 8 mol) wurden bei 30 bis 35°C unter Rühren 445,7 g Acrylnitril (entsprechend 8,4 mol) innerhalb einer Stunde zugetropft. Anschließend ließ man drei Stunden bei 30°C nachreagieren. In der letzten Stunde wurde ein kräftiger Stickstoffstrom durch die Reaktionslösung geleitet und es wurden insgesamt ca. 100 g Acrylnitril/Wasser-Gemisch kondensiert, davon ca. 21 g Acrylnitril.

Danach wurden bei 40°C unter Rühren 648 g 50 gew.-%ige Natronlauge (entsprechend 8,1 mol) innerhalb von 30 Minuten zugetropft. Nach vier Stunden bei 40°C wurde die Temperatur für weitere 8 Stunden auf 60°C erhöht. Während der Natronlauge-Zugabe und der Reaktionszeit wurde ein kräftiger Luftstrom durch die Reaktionslösung geleitet und es wurden insgesamt ca. 600 ml Ammoniakwasser abdestilliert bis die Ammoniak-Konzentration im Reaktionsgemisch einen Wert von 100 ppm erreichte. Die so erhaltene Lösung wurde mit Wasser auf eine Konzentration von 40 Gew.-% an β-Alanindiessigsäure-tri-Natriumsalz eingestellt.

Eine repräsentative Probe wurde im Vakuum bis zur Gewichtskonstanz getrocknet und analytisch untersucht:
ADA-Na₃: 99,6 %
Gehalt an Iminodiessigsäure: 0,3 %
Gehalt an Acrylsäure: < 0,05 %
Gehalt an Acrylnitril: < 20 ppm

### Beispiel 2

### Herstellung von β-Alanindiessigsäure. (Acrylnitril-Weg)

Eine 40 gew.-%ige wäßrige β-Alanindiessigsäure-tri-Natriumsalz-Lösung wurde nach Beispiel 1 hergestellt. Zu 1 kg dieser Lösung wurden bei 20 bis 40°C unter Rühren 434 g 50 gew.-%ige Schwefelsäure zugetropft, wobei sich ein pH-Wert von ca. 2,0 einstellte. Anschließend wurde auf 20°C abgekühlt und eine Stunde bei dieser Temperatur nachgerührt. Der ausgefallene Niederschlag wurde abfiltriert, mit Eiswasser gewaschen, bis im Waschwasser der Sulfatnachweis mit wäßriger Bariumchlorid-Lösung negativ ausfiel, und im Vakuum bei 60°C getrocknet. Man erhielt 284 g der Titelverbindung, entsprechend einer Ausbeute von 94 %; das Produkt schmolz bei 205°C unter Zersetzung und hatte eine Reinheit von 99,9 %.
Gehalt an Iminodiessigsäure: < 0,1 %
Gehalt an Acrylsäure: < 0,05 %
Gehalt an Sulfat: < 0,1 ppm

### Beispiel 3

### Herstellung von β-Alanindiessigsäure-tri-Natrium-Salz (Acrylester-Weg)

Zu 3540 g einer 40 gew.-%igen wäßrigen Lösung des Dinatriumsalzes von Iminodiessigsäure (entsprechend 8 mol) wurden bei 20 bis 30°C unter Rühren 723,2 g Acrylsäuremethylester (entsprechend 8,4 mol) innerhalb einer Stunde zugetropft. Anschließend ließ man vier Stunden bei 30°C nachreagieren. In der letzten Stunde wurde ein kräftiger Stickstoffstrom durch die Reaktionslösung geleitet und es wurden insgesamt ca. 160 g Acrylsäuremethylester/Wasser-Gemisch kondensiert, davon ca. 34 g Acrylsäuremethylester.

Zur Verseifung des Esters wurden bei 40°C unter Rühren 648 g 50 gew.-%ige Natronlauge (entsprechend 8,1 mol) innerhalb von 30 Minuten zugetropft. Die Reaktionslösung wurde weitere zehn Stunden bei 40°C gehalten. Während der Natronlauge-Zugabe und der Reaktionszeit wurde ein kräftiger Luftstrom durch die Reaktionslösung geleitet und es wurden insgesamt ca. 250 ml Methanol und ca. 550 ml Wasser abdestilliert. Die so erhaltene Lösung wurde mit Wasser auf einen Gehalt von 40 Gew.-% an β-Alanindiessigsäure-tri-Natriumsalz eingestellt.

Eine repräsentative Probe wurde im Vakuum bis zur Gewichtskonstanz getrocknet und analytisch untersucht:
ADA-Na₃: 99,6 %
Gehalt an Iminodiessigsäure: 0,2 %
Gehalt an Acrylsäure: < 0,05 %
Gehalt an Acrylsäuremethylester: < 20 ppm
Gehalt an Methanol: < 50 ppm

### Beispiel 4

### Herstellung von β-Alanindiessigsäure (Acrylester-Weg)

Eine 40 gew.-%ige wäßrige β-Alanindiessigsäure-tri-Natriumsalz-Lösung wurde nach Beispiel 3 hergestellt. Zu 1 kg dieser Lösung wurden bei 20 bis 40°C unter Rühren 434 g 50 gew.-%ige Schwefelsäure zugetropft, wobei sich ein pH-Wert von ca. 2,0 einstellte. Anschließend wurde auf 20°C abgekühlt und eine Stunde bei dieser Temperatur nachgerührt. Der ausgefallene Niederschlag wurde abfiltriert, mit Eiswasser gewaschen, bis im Waschwasser der Sulfatnachweis mit wäßriger Bariumchlorid-Lösung negativ ausfiel, und im Vakuum bei 60°C getrocknet. Man erhielt 363 g der Titelverbindung, entsprechend einer Ausbeute von 98 %; das Produkt schmolz bei 204 - 205°C unter Zersetzung und hatte eine Reinheit von 99,9 %.
Gehalt an Iminodiessigsäure: < 0,1 %
Gehalt an Acrylsäure: < 0,05 %
Gehalt an Acrylsäuremethylester: < 0,01 ppm
Gehalt an Sulfat: < 0,1 %

### Beispiel 5

### Herstellung von β-Alanindiessigsäure-di-Natrium-salz (Acrylester-Weg)

1064 g Iminodiessigsäure (entsprechend 8 mol) wurden in 2000 g Wasser suspendiert und mit 640 g 50 gew.-%iger Natronlauge versetzt (ca. pH 7,0). Dabei ging die Iminodiessigsäure in Lösung. Bei 20 bis 30°C wurden unter Rühren 723,2 g Acrylsäuremethylester (entsprechend 8,8 mol) innerhalb einer Stunde zugetropft. Anschließend ließ man zwölf Stunden bei 30°C nachreagieren. In der letzten Stunde wurde ein kräftiger Stickstoffstrom durch die Reaktionslösung geleitet und es wurden insgesamt ca. 340 g Acrylsäuremethylester/Wasser-Gemisch kondensiert, davon ca. 68 g Acrylsäuremethylester.

Anschließend wurden bei 40°C unter Rühren 648 g 50 gew.-%ige Natronlauge (entsprechend 8,1 mol) innerhalb von 30 Minuten zugetropft und es wurde weitere zehn Stunden bei 40°C gerührt. Während der Reaktionszeit wurde ein kräftiger Luftstrom durch die Reaktionslösung geleitet und es wurden insgesamt ca. 800 g Methanol/Wasser-Gemisch, davon ca. 250 g Methanol, abdestilliert. Die so erhaltene Lösung wurde mit Wasser auf einen Gehalt von 40 Gew.-% an β-Alanindiessigsaure-di-Natriumsalz eingestellt.

Eine repräsentative Probe wurde im Vakuum bis zur Gewichtskonstanz getrocknet und analytisch untersucht:
ADA-Na₂: 99,6 %
Gehalt an Iminodiessigsäure: 0,2 %
Gehalt an Acrylsäure: < 0,05 %
Gehalt an Acrylsäuremethylester: < 20 ppm
Gehalt an Methanol: < 50 ppm

### Beispiel 6

### Herstellung von β-Alanindiessigsäure-tri-Natrium-salz (Acrylester-Weg)

Zu 708 g einer 20 gew.-%igen wäßrigen Lösung des Dinatriumsalzes von Iminodiessigsaure (entsprechend 0,8 mol) wurden bei 20 bis 30°C unter Rühren auf einmal 50 g Ethanol und anschließend 84,1 g Acrylsäureethylester (entsprechend 0,84 mol) innerhalb einer Stunde zugetropft. Anschließend ließ man fünf Stunden bei 30°C nachreagieren. In den letzten beiden Stunden wurde bei 800 mbar und 30°C ein kräftiger Stickstoffstrom durch die Reaktionslösung geleitet und es wurden insgesamt ca. 135 g Acrylsäureethylester/Ethanol/Wasser-Gemisch kondensiert, davon ca. 4,0 g Acrylsäureethylester und 50 g Ethanol. Danach wurden bei 40°C unter Rühren 64,8 g 50 gew.-%ige Natronlauge (entsprechend 0,81 mol) innerhalb von 30 Minuten zugetropft und die Reaktionslösung wurde weitere zehn Stunden bei 40°C gehalten. Während der Natronlauge-Zugabe und der Reaktionszeit wurde ein kräftiger Luftstrom durch die Reaktionslösung geleitet und es wurden insgesamt ca. 36,8 g Ethanol und ca. 85 g Wasser abdestilliert. Die so erhaltene Losung wurde mit Wasser auf einen Gehalt von 20 Gew.-% an β-Alanindiessigsäure-tri-Natriumsalz eingestellt.

Eine repräsentative Probe wurde im Vakuum bis zur Gewichtskonstanz getrocknet und analytisch untersucht:
ADA-Na₃: 99,6 %
Gehalt an Iminodiessigsäure: 0,2 %
Gehalt an Acrylsäure: < 0,05 %
Gehalt an Acrylsäureethylester: < 20 ppm
Gehalt an Ethanol: < 50 ppm
Gehalt an Sulfat: < 0,1 %

## Patentansprüche

1. Verfahren zur Herstellung von β-Alanindiessigsäure (Ia) oder ihren Alkalimetall- oder Ammoniumsalzen (Ib) dadurch gekennzeichnet, daß man Iminodiessigsäure mit Acrylnitril oder C₁- bis C₄-Alkylacrylsäureestern im schwach sauren bis schwach basischen wäßrigen Medium miteinander umsetzt und anschließend die Nitril- oder Estergruppe zur Säure oder einem Salz verseift.

2. Verfahren gemäß Anspruch 1, dadurch gekennzeichnet, daß man bei der Additionsreaktion Temperaturen ≤ 60°C einhält.

3. Verfahren gemäß Anspruch 1, dadurch gekennzeichnet, daß man bei pH-Werten von 5 bis 10, vorzugsweise 6 bis 8 arbeitet.

## Claims

1. A process for preparing β-alaninediacetic acid (Ia) or its alkali metal or ammonium salts (Ib) which comprises reacting iminodiacetic acid with acrylonitrile or C₁-C₄-alkyl acrylic esters in weakly acid to weakly basic aqueous medium and subsequently hydrolyzing the nitrile or ester group to the acid or a salt.

2. A process as claimed in claim 1, wherein the temperature in the addition reaction is kept at ≤60°C.

3. A process as claimed in claim 1, which is carried out at a pH of from 5 to 10, preferably 6 to 8.

## Revendications

1. Procédé de préparation d'acide β-alanine-diacétique (Ia) ou de ses sels de métaux alcalins ou d'ammonium (Ib), caractérisé en ce que l'on fait réagir entre eux de l'acide iminodiacétique et de l'acrylonitrile ou des esters alkyliques en C₁-C₄ d'acide acrylique en milieu aqueux faiblement acide à faiblement basique, et on saponifie ensuite le groupement nitrile ou ester pour obtenir l'acide ou un sel.

2. Procédé selon la revendication 1, caractérisé en ce que l'on maintient une température inférieure ou égale à 60°C au cours de la réaction d'addition.

3. Procédé selon la revendication 1, caractérisé en ce que l'on procède à un pH de 5 à 10, de préférence de 6 à 8.
